# EUROPÄISCHE PATENTSCHRIFT

(11) **EP 3 427 715 B1**
(45) Veröffentlichungstag und Bekanntmachung des Hinweises auf die Patenterteilung: **03.03.2021**
(21) Anmeldenummer: 17181491.6
(22) Anmeldetag: 14.07.2017
(51) Int. Cl.: A61K 6/842, A61K 6/853, A61K 6/887

(54) **DENTALWERKSTOFFE AUF BASIS DÜNNFLÜSSIGER RADIKALISCH POLYMERISIERBARER MONOMERE MIT HOHEM BRECHUNGSINDEX**
DENTAL MATERIALS BASED ON LOW VISCOSITY RADICALLY POLYMERIZABLE MONOMERS HAVING A HIGH REFRACTIVE INDEX
MATIÈRE DENTAIRE À BASE DE MONOMÈRE FLUIDE POUVANT ÊTRE DURCI PAR POLYMÉRISATION RADICALAIRE AYANT UN INDICE DE RÉFRACTION ÉLEVÉ

(43) Veröffentlichungstag der Anmeldung: 16.01.2019
(73) Patentinhaber: Ivoclar Vivadent AG, 9494 Schaan (LI)
(72) Erfinder: Moszner, Norbert, 9493 Mauren (LI); Fischer, Urs Karl, 9320 Arbon (CH); Fässler, Pascal, 7320 Sargans (CH)
(74) Vertreter: Uexküll & Stolberg

(56) Entgegenhaltungen:
- JP-A- H08 157 320
- US-A- 5 183 917
- US-A1- 2011 026 118

## Beschreibung

Die vorliegende Erfindung betrifft Dentalwerkstoffe auf Basis dünnflüssiger radikalisch polymerisierbarer Monomere mit hohem Brechungsindex, die sich besonders als dentale Zemente und Füllungskomposite und zur Herstellung von Formkörpern eignen.

Dentalmaterialien, die z.B. als Zement oder als direktes Füllungsmaterial eingesetzt werden, enthalten in der Regel eine polymerisierbare organische Matrix und einen oder mehrere Füllstoffe, die meist mit einem polymerisationsfähigen Haftvermittler oberflächenmodifiziert sind. Der Füllstoffgehalt hängt maßgeblich vom gewünschten Verwendungszweck ab und kann bis zu 90 Gew.-% betragen, wobei Befestigungszemente im Vergleich zu Füllungsmaterialien einen geringeren Füllungsgrad aufweisen. Die polymerisierbare organische Matrix enthält gewöhnlich eine Mischung von Monomeren, Initiatorkomponenten, Stabilisatoren und Pigmenten. Dentalmaterialien, die eine polymerisierbare Matrix und Füllstoff enthalten, werden als Komposite bezeichnet. Die polymerisierbare Matrix wird auch als Harz bezeichnet.

Als Monomere werden meist Mischungen von Dimethacrylaten eingesetzt. Verbreitete Beispiele hierfür sind die hochviskosen Dimethacrylate 2,2-Bis[4-(2-hydroxy-3-methacryloyloxypropyl)phenyl]propan (Bis-GMA) und 1,6-Bis-[2-methacryloyloxy-ethoxycarbonylamino]-2,2,4-trimethylhexan (UDMA) und die als Verdünnermonomere genutzten niedrigerviskosen Dimethacrylate Bis(3-methacryloyloxymethyl)tricyclo-[5.2.1.0^{2,6}]decan (TCP), Decandiol-1,10-dimethacrylat (D₃MA) und Triethylenglycoldimethacrylat (TEGDMA). Dimethacrylate bewirken bei der Polymerisation eine dreidimensionale Vernetzung der sich bildenden Polymerketten und ergeben damit eine verbesserte mechanische Stabilität.

Die Materialen enthalten gewöhnlich auch einen Initiator für die radikalische Polymerisation, wobei lichthärtende Werkstoffe, die einen Photoinitiator enthalten, in der zahnärztlichen Füllungstherapie heutzutage eine dominante Stellung einnehmen.

Ein Nachteil von lichthärtenden Materialien ist, dass insbesondere das Legen großer Füllungen mit einem erheblichen Aufwand verbunden ist, weil das zur Härtung erforderliche Licht nur bis zu einer begrenzten Tiefe in die Materialien eindringen kann. Bei der sogenannten Inkrementtechnik wird die Füllung daher schichtweise aus dem Kompositwerkstoff aufgebaut, wobei die Schichten eine Dicke von jeweils etwa 2 mm haben und einzeln gehärtet werden.

Neuerdings finden sogenannte "Bulk-Fill"-Komposite, die Schichtdicken von 4 bis 5 mm erlauben, aufgrund der möglichen Zeitersparnis großes Interesse. Voraussetzung für die klinische Eignung dieser Materialien ist eine große Durchhärtungstiefe.

Die Durchhärtungstiefe ist sowohl von Prozessparametern als auch von den Materialeigenschaften abhängig. So besteht z.B. zwischen der Durchhärtungstiefe und der Intensität des eingestrahlten Lichtes bzw. der Belichtungszeit ein logarithmischer Zusammenhang. Weiterhin korreliert die Durchhärtungstiefe mit der Transparenz und Transluzenz der Materialien. Üblicherweise spricht man bei füllstoffhaltigen Materialien von Transluzenz statt von Transparenz (Durchsichtigkeit), weil sie Licht zwar durchlassen, in der Regel aber nicht transparent sind. Als transluzent werden in der Technik Körper mit einer Lichtdurchlässigkeit von kleiner als 90% bezeichnet.

Die Lichtdurchlässigkeit von Polymeren ist im Idealfall (keine Absorption und Brechung) von deren Brechungsindex n abhängig und variiert zwischen 98,4 % (n = 1,29) und 92,8 % (n = 1,73) (vgl. H.-G. Elias, Makromoleküle - Anwendungen von Polymeren, Bd. 4. 6. Auflage, Wiley-VCH, Weinheim 2003, 517). Diese Idealwerte werden jedoch nur selten erreicht, weil das Licht gestreut und absorbiert wird.

Die Lichtdurchlässigkeit von Kompositen wird u.a durch die Brechungsindices der Harzmatrix und der Füllstoffe, durch die Größe der Füllstoffpartikel (Streuung) sowie die Art und Konzentration zugesetzter Farbmittel (Absorption) beeinflusst. Der Brechungsindex besonders häufig eingesetzter Dentalmonomere nimmt in folgender Reihenfolge ab: Bis-GMA (1,5512), ethoxyliertes Bis-GMA (SR-348c) (1,5393), UDMA (1,4850), TEGDMA (1,4610) und D₃MA (1,4600). Der Brechungsindex der als Füllstoff häufig verwendeten röntgenopaken Gläser liegt im Bereich von 1,523-1,550 und der Brechungsindex von Zahnschmelz bei 1,655.

Bei Kompositen kann eine hohe Lichtdurchlässigkeit und damit eine gute Durchhärtungstiefe dadurch erreicht werden, dass eine organische Matrix und Füllstoffe mit übereinstimmenden Brechungsindices verwendet werden. Aromatische Methacrylate haben häufig hohe Brechungsindices, die annähernd den Brechungsindices röntgenopaker Gläser entsprechen. Andererseits weisen solche Monomere oft eine hohe Viskosität auf und lassen sich erst nach Zugabe von niedrigviskosen Monomeren verarbeiten und mit Füllstoffen mischen. Die im Dentalbereich üblicherweise eingesetzten Verdünnermonomere wie TEGDMA oder D₃MA haben aber nur relative niedrige Brechungsindices, meist deutlich unter 1,50, so dass ihre Zugabe eine Verringerung des Brechungsindex der Harzmischung und damit eine Beeinträchtigung der Lichtdurchlässigkeit bewirkt.

Die JP H08-157320 offenbart Dentalwerkstoffe, die schwefelhaltige (Meth)acrylester mit einem Brechungsindex von 1,56 oder mehr enthalten. Die Werkstoffe sollen sich durch eine schnelle Härtung auszeichnen, und die erhaltenen Polymere sollen nicht trübe sein.

Die US 2011/0026118 A1 offenbart lichtbrechende optische Elemente, die zwei Schichten mit unterschiedlichen Brechungsindices aufweisen. Mindestenes eine Schicht wird durch ein gehärtetes optisches Adhäsiv gebildet, das vorzugsweise auf einem Epoxidharz oder einem Thiol-En-System basiert, wie z.B. Bis(4-vinylthiophenyl)sulfid.

Die US 5, 183,917 offenbart Zusammensetzungen auf der Basis von 4,4'-Bis(methacryloylthio)diphenylsulfid und damit co-polymerisierbaren Vinylmonomeren, die einen hohen Brechungsindex aufweisen und sich insbesondere als optische Materialien eignen sollen.

Der Erfindung liegt die Aufgabe zugrunde, lichthärtbare Werkstoffe zur Verfügung zu stellen, die sich insbesondere für dentale Zwecke eignen und die eine hohe Durchhärtungstiefe und einen geringen Polymerisationsschrumpf aufweisen.

Diese Aufgabe wird erfindungsgemäß durch Werkstoffe gelöst, die mindestens ein radikalisch polymerisierbares, difunktionelles Phenylensulfid gemäß der allgemeinen Formeln I enthalten, 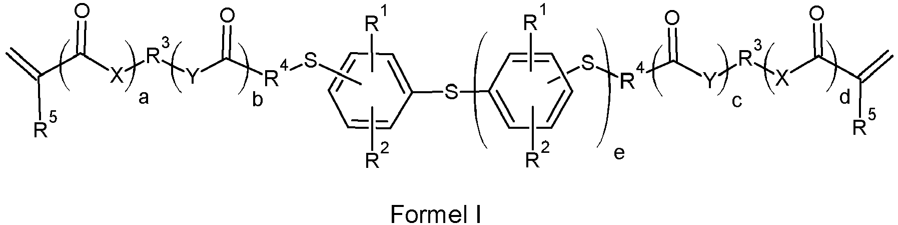 in der
- X, Y: unabhängig voneinander jeweils entfallen oder O oder NR⁶ sind,
- R¹, R²: unabhängig voneinander jeweils H, ein linearer oder verzweigter C₁- bis C₁₀-Alkylrest, -OR⁷, -SR⁸, Cl oder Br sind,
- R³: entfällt oder ein C₁-C₁₀-Alkylenrest ist, der durch O oder S unterbrochen sein kann,
- R⁴: entfällt oder ein C₁-C₁₀-Alkylenrest ist, der durch O oder S unterbrochen sein kann,
- R⁵: H oder ein C₁-C₅-Alkylrest ist,
- R⁶, R⁷, R⁸: unabhängig voneinander jeweils H oder ein linearer oder verzweigter C₁- bis C₁₀-Alkylrest sind,
- a, b, c, d: unabhängig voneinander jeweils 0 oder 1 sind und
- e: 1 oder 2 ist, und
mindestens ein weiteres radikalisch polymerisierbares Monomer enthalten, das aus 2,2-Bis[4-(2-hydroxy-3-methacryloyloxypropyl)phenyl]propan (Bis-GMA), UDMA (ein Additionsprodukt aus 2-Hydroxyethylmethacrylat und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat), Decandiol-1,10-dimethacrylat (D₃MA), Triethylenglycoldimethacrylat (TEGDMA), p-Cumylphenoxyethylenglycolmethacrylat (CMP-1E), Bis(3-methacryloyloxymethyl)-tricyclo-[5.2.1.0^{2,6}]decan (TCP) und 2-[4-(2-Methacryloyloxyethoxyethoxy)phenyl]-2-[4-(2-methacryloyloxyethoxy)phenyl]propan) ausgewählt ist.

Bevorzugt sind Verbindungen, in denen a = d und b = c ist. Wenn R³ entfällt, entfällt vorzugsweise auch X oder Y, besonders bevorzugt entfallen X und Y.

Die Formel I erstreckt sich nur auf solche Verbindungen, die mit der chemischen Valenzlehre vereinbar sind. Der Hinweis, dass ein Rest durch ein oder mehrere O-Atome oder S-Atome unterbrochen ist, ist so zu verstehen, dass diese Atome jeweils in die Kohlenstoffkette des Restes eingeschoben werden. Diese Atome sind damit beidseitig durch C-Atome begrenzt und können nicht endständig sein. C₁-Reste können nicht unterbrochen sein.

Bevorzugt sind difunktionelle Phenylensulfide der Formel I, wobei mindestens eine Variable eine der folgenden Bedeutungen hat:
- X, Y: unabhängig voneinander jeweils entfällt oder O,
- R¹, R²: unabhängig voneinander jeweils H oder Br, CH₃, SCH₃, OCH₃,
- R³: entfällt oder ein C₂-C₄-Alkylenrest,
- R⁴: entfällt oder ein C₁-C₃-Alkylenrest,
- R⁵: H oder CH₃,
- a, b, c, d: unabhängig voneinander jeweils 0 oder 1, und
- e: 1.

Vorzugweise haben alle Variablen die bevorzugten Bedeutungen.

Besonders bevorzugt sind difunktionelle Phenylensulfide der Formel I, wobei mindestens eine Variable eine der folgenden Bedeutungen hat:
- R¹, R²: unabhängig voneinander jeweils H oder Br,
- R³: entfällt oder ein C₂-C₄-Alkylenrest,
- R⁴: entfällt oder ein C₁-C₃-Alkylenrest,
- R⁵: H,
- a, b, c, d: jeweils 0, und
- e: 1.

Vorzugweise haben alle Variablen die besonders bevorzugten Bedeutungen.

Ganz besonders bevorzugt sind in allen Fällen solche Verbindungen, in denen die Vinylsulfidgruppen jeweils in p-Position zu dem S-Atom an die Phenylringe gebunden sind, das die Phenylringe verbrückt.

Die erfindungsgemäß verwendeten difunktionellen Phenylensulfide der allgemeinen Formeln I sind auf an sich bekannte Weise zugänglich. Beispielsweise lässt sich das besonders bevorzugte Bis(4-vinylthiophenyl)sulfid aus dem kommerziell zugänglichen 4,4'-Thiobis(benzolthiol) herstellen. Dabei erfolgt zunächst die Thioetherbildung durch Umsetzung mit überschüssigem 1,2-Dichlorethan in Gegenwart von KOH und Tetrabutylammoniumchlorid bei Raumtemperatur, wobei sich das entsprechende Bis[4-(2-chlorethylthiophenyl)sulfid bildet, dessen Dehydrohalogenierung mit KOH in Wasser bei erhöhter Temperatur, vorzugsweise bei 85 °C, zum Bis(4-vinylthiophenyl)sulfid führt:

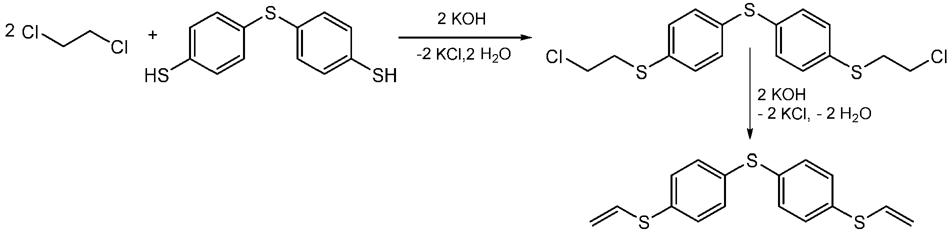

Bevorzugte difunktionelle Phenylensulfide der allgemeinen Formel I sind:

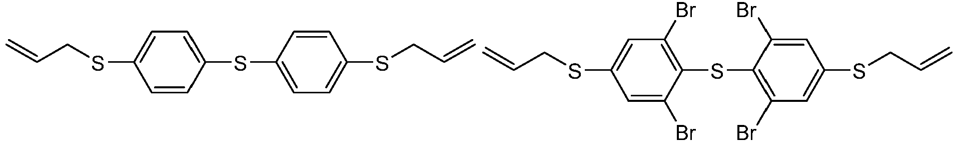

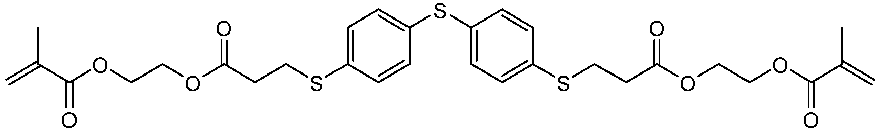

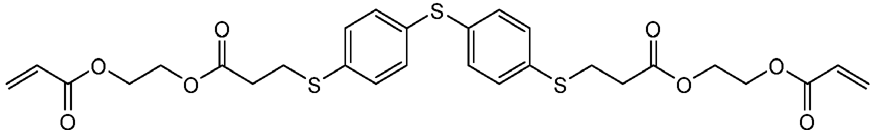

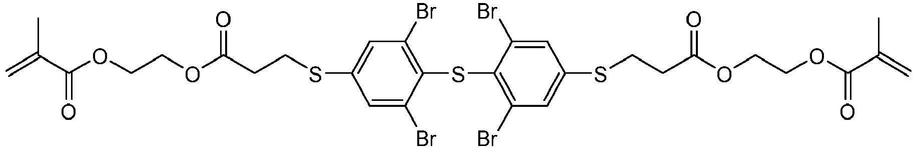

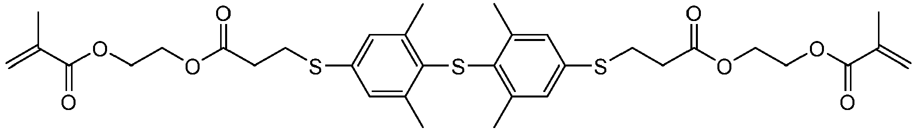

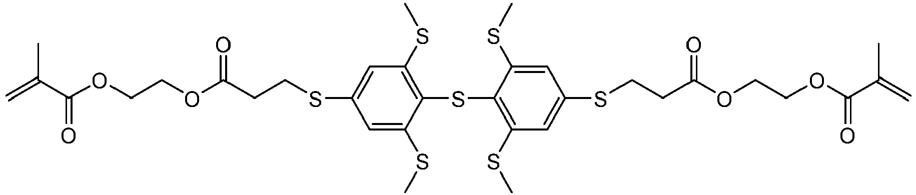

Besonders bevorzugte difunktionelle Phenylensulfide der allgemeinen Formel I sind:

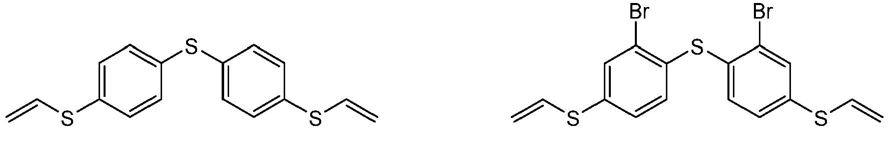

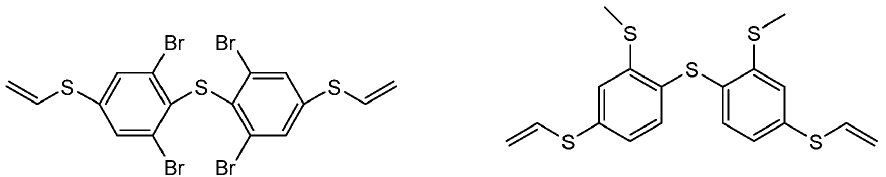

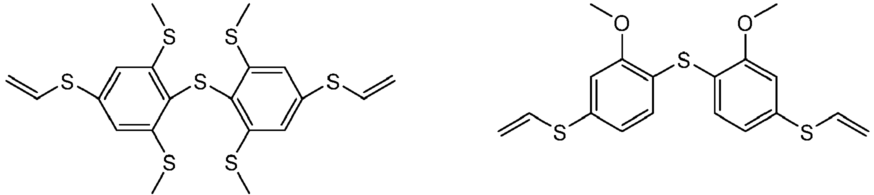

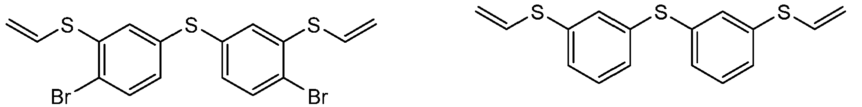

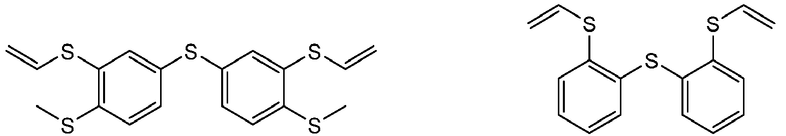

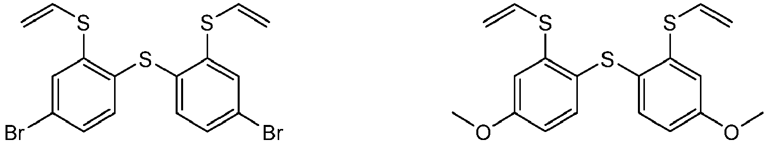

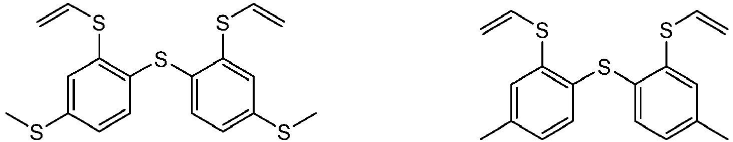

Die Monomere der Formel I zeichnen sich durch eine geringe Viskosität aus. Sie haben vorzugsweise eine Viskosität ≤ 3 Pa·s, vorzugsweise 5 bis 2.000 mPa·s und besonders bevorzugt 10 bis 1.000 mPa·s, wobei die Viskosität mit einem Kapillarviskosimeter bei einer Temperatur von 23°C bestimmt wird.

Die erfindungsgemäß verwendeten difunktionellen Phenylensulfide der Formel I werden in Mischung mit mindestens einem weiteren radikalisch polymerisierbaren Monomer zur Herstellung von Dentalwerkstoffen eingesetzt. Die Werkstoffe enthalten als radikalisch polymerisierbares Monomer mindestens ein mono- oder multifunktionelles (Meth)acrylat, das aus den oben genannten Monomeren ausgewählt ist. Unter monofunktionellen (Meth)acrylaten werden Verbindungen mit einer, unter multifunktionellen (Meth)¬acrylaten Verbindungen mit zwei oder mehr, vorzugsweise 2 bis 4 radikalisch polymerisierbaren Gruppen verstanden. Gemäß einer bevorzugten Ausführungsform enthalten die erfindungsgemäßen Zusammensetzungen mindestens ein Dimethacrylat oder eine Mischung von Mono- und Dimethacrylaten. Materialien, die intraoral gehärtet werden sollen, enthalten als radikalisch polymerisierbares Monomer vorzugsweise mono- und/oder multifunktionelle Methacrylate.

Beispiele für mono- oder multifunktionelle (Meth)acrylate sind Methyl-, Ethyl-, 2-Hydroxyethyl-, Butyl-, Benzyl-, Tetrahydrofurfuryl- und Isobornylmethacrylat, p-Cumylphenoxyethylenglycolmethacrylat (CMP-1E), Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, wie z.B. 2-[4-(2-Methacryloyloxyethoxyethoxy)phenyl]-2-[4-(2-methacryloyloxyethoxy)phenyl]propan) (SR-348c, Firma Sartomer; enthält 3 Ethoxygruppen) und 2,2-Bis[4-(2-methacryloxy-propoxy)phenyl]propan, UDMA (ein Additionsprodukt aus 2-Hydroxyethylmethacrylat und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat), Di-, Tri- oder Tetraethylenglycoldimethacrylat, Trimethylolpropantrimethacrylat, Pentaerythrittetramethacrylat, sowie Glycerindi- und Glycerintrimethacrylat, 1,4-Butandioldimethacrylat, 1,10-Decandioldimethacrylat (D₃MA), 1,12-Dodecandioldimethacrylat, TCP und Mischungen davon. Es wurde überraschend gefunden, dass sich die difunktionellen Phenylensulfide der allgemeinen Formeln I gut mit üblichen Dentalmonomeren und insbesondere mit mono- oder multifunktionellen (Meth)acrylaten und ganz besonders mit den oben genannten (Meth)acrylaten copolymerisieren lassen. Dies war nicht zu erwarten, weil die difunktionellen Phenylensulfide der Formel I π-elektronenreiche Monomere darstellen, die sich bekanntlich nur schlecht mit (Meth)acrylaten radikalisch copolymerisieren lassen. Die Copolymerisierbarkeit ist eine wesentliche Voraussetzung zur Herstellung von Dentalwerkstoffen.

Die difunktionellen Phenylensulfide der Formel I zeichnen sich außerdem durch einen geringen Polymerisationsschrumpf aus. Dieser liegt unter 10 Vol.-%, was für dentale Anwendungen ebenfalls ein wesentliches Kriterium ist.

Zur Herstellung von Kompositen sind erfindungsgemäß Monomere und Monomermischungen bevorzugt, die einen Brechungsindex haben, der dem des verwendeten Füllstoffs nahekommt und damit eine hohe Transluzenz erwarten lässt. Dentale Füllstoffe haben meist einen Brechungsindex von etwa 1,523 bis 1,550. Das weit verbreitete Bis-GMA weist diesbezüglich zwar einen sehr vorteilhaften Brechungsindex auf (n_{D} = 1,5512), seine hohe Viskosität erschwert jedoch das Einarbeiten von Füllstoffen und schränkt die maximale Füllstoffmenge stark ein. Übliche Verdünnermonomere haben meist einen Brechungsindex von deutlich unter 1,50 und bewirken daher neben der angestrebten Verringerung der Viskosität auch eine unerwünschte Absenkung des Brechungsindexes der Monomermischung. Die erfindungsgemäß verwendeten difunktionellen Phenylensulfide der allgemeinen Formeln I zeichnen sich demgegenüber durch eine geringe Viskosität und einen hohen Brechungsindex aus. Sie ermöglichen daher eine Einstellung der Viskosität der Monomermischung, ohne den Brechungsindex zu verringern, bzw. eine Einstellung des Brechungsindexes ohne die Viskosität zu erhöhen.

Erfindungsgemäß bevorzugte Verbindungen der Formel I haben einen Brechungsindex von mehr 1,57, vorzugsweise von 1,575 bis 1,75 und besonders bevorzugt von 1,58 bis 1,72.

Der Brechungsindex ist eine Stoffkonstante, die von der Wellenlänge des verwendeten Lichts, von der Temperatur, vom Druck und der Reinheit des Stoffes abhängt. Wenn nicht anders angegeben wird hier unter dem Brechungsindex der bei 20°C mit dem Licht der gelben Na-D-Linie (λ = 589 nm) gemessene Brechungsindex verstanden (n_{D}²⁰ oder kurz n_{D}). Die Bestimmung des Brechungsindex flüssiger Monomere und Monomermischungen kann mit einem handelsüblichen Abbe-Refraktometer erfolgen.

Die erfindungsgemäßen Werkstoffe enthalten vorzugsweise mindestens einen Photoinitiator für die radikalische Polymerisation, besonders Photoinitiator, der in einem Wellenlängenbereich von 400 bis 500 nm aktiv ist.

Bevorzugte Photoinitiatoren sind Photosensibilisatoren, vor allem von α-Diketone, wie 9,10-Phenanthrenchinon, 1-Phenyl-propan-1,2-dion, Diacetyl oder 4,4'-Dichlorbenzil oder deren Derivate, besonders bevorzugt Campherchinon (CC) und dessen Derivate, und Mischungen davon.

Die Photoinitiatoren werden vorzugsweise in Kombination mit Beschleunigern eingesetzt. Als Beschleuniger eignen sich besonders tertiäre Amine, wie z.B. tertiäre aromatische Amine, insbesondere N,N-Dialkylaniline, -p-toluidine oder -3,5-xylidine, p-(N,N-Dialkylamino)-phenylethanol, -benzoesäurederivate, -benzaldehyd, -phenylessigsäureester und -phenylpropionsäureester. Konkrete Beispiele hierfür sind N,N-Dimethylanilin, N,N-Dimethyl-p-toluidin, N,N,3,5-Tetramethylanilin, N,N-Dimethyl-amino-p-benzaldehyd, p-(Dimethylamino)-benzoesäureethylester oder p-(Dimethyl-amino)-benzonitril. Geeignet sind auch tertiäre aliphatische Amine, wie z.B. Tri-n-butylamin, Dimethylaminoethan-2-ol, Triethanolamin, Dimethylaminoethylmethacrylat, N,N-Dimethylbenzylamin, oder heterocyclische Amine, wie z.B. 1,2,2,6,6-Pentamethylpiperidin, und Aminosäure-Derivate, wie z.B. N-Phenylglycin.

In Zusammensetzungen, die acide Monomere enthalten, wie z.B. selbsthaftenden Kompositen, werden vorzugsweise aminfreie Beschleuniger verwendet, wie z.B. Sulfinsäuren und Sulfinate, Borate, Enolate, Phosphine oder andere Verbindungen, die aktive Wasserstoffatome enthalten, z.B. heterocylische Verbindungen wie Morpholin-Derivate oder 1,3-Dioxolane.

Besonders bevorzugte Photoinitiatoren sind Acyl- oder Bisacylgermanium-Verbindungen, insbesondere die in der EP 1 905 413 A1 offenbarten Monoacyltrialkyl- und Bisacyldialkylgermanium-Verbindungen, wie z.B. Benzoyltrimethylgermanium, Bisbenzoyldiethylgermanium oder Bis(4-methoxybenzoyl)diethylgermanium. Acyl- und Bisacylgermanium-Verbindungen haben den Vorteil, dass sie sich nach der Bestrahlung entfärben (Bleaching Effekt) und so die Transparenz der ausgehärteten Werkstoffe nicht beeinträchtigen. Außerdem handelt es sich um monomolekulare Photoinitiatoren, d.h. sie benötigen keinen Beschleuniger, um ihre volle Aktivität zu erreichen.

Weitere besonders bevorzugte Photoinitiatoren sind Acyl- oder Bisacylphosphinoxide, insbesondere die in EP 0 007 505, EP 0 073 413, EP 0 184 095 und EP 0 615 980 beschriebenen Verbindungen. Bevorzugte Beispiele sind die kommerziell zugänglichen Verbindungen 2,4,6-Trimethylbenzoyldiphenylphosphinoxid (Lucirin® TPO, BASF) und Bis(2,4,6-trimethylbenzoyl)phenylphosphinoxid (Irgacure® 819, Ciba). Acyl- und Bisacylphosphinoxide gehören ebenfalls zur Gruppe der monomolekularen Photoinitiatoren und zeichnen sich durch eine geringe Eigenabsorption aus.

Die erfindungsgemäßen Werkstoffe können zusätzlich einen oder mehrere weitere Initiatoren für die radikalische Polymerisation enthalten, wie z.B. Peroxide oder Hydroperoxide oder Redox-Initiatorkombinationen, wie z.B. Kombinationen von Benzoylperoxid mit N,N-Dimethyl-sym.-xylidin oder N,N-Dimethyl-p-toluidin, Kombinationen von anorganischen Peroxiden, vor allem Kalium- und Ammoniumperoxodisulfat, mit Reduktionsmitteln wie, Sulfit, Hydrogensulfit, Thiosulfat, Sulfinsäuren, Aminen, Endiolen oder Fe-(II)-Salzen, Redoxsysteme bestehend aus organischen Peroxiden bzw. Hydroperoxiden und solchen Reduktionsmitteln, wie z.B. Ascorbinsäure, Barbiturate, Thioharnstoffe oder Sulfinsäuren. Weiterhin lassen sich als Übergangsmetall-Redoxkatalysatoren Verbindungen von Übergangsmetallen, die mindestens 2-stabile Wertigkeitsstufen aufweisen, einsetzen. Das sind vor allem Verbindungen der Elemente Kupfer, Eisen, Vanadium, Nickel oder Kobalt, wobei Kupferverbindungen besonders bevorzugt sind und diese bevorzugt als gut organolösliche Verbindungen, wie z.B. als Acetylacetonat, Naphthenat oder 2-Ethyl-hexanoat eingesetzt werden.

Die erfindungsgemäßen Dentalwerkstoffe können organische oder vorzugsweise anorganische oder organisch-anorganische Füllstoffe enthalten, wobei partikuläre Füllstoffe bevorzugt sind. Bevorzugte anorganische partikuläre Füllstoffe sind Pulver röntgenopaker Gläser mit einer durchschnittlichen Teilchengröße von 0,01 bis 15 µm, vorzugsweise 0,10 bis 5,0 µm; röntgenopake Füllstoffe, wie Ytterbiumtrifluorid, mit einer durchschnittlichen Teilchengröße von 0,050 bis 2,0 µm, vorzugsweise 0,10 bis 1,0 µm; Mischoxide aus SiO₂, ZrO₂, ZnO und/oder TiO₂ mit einer durchschnittlichen Teilchengröße von 5 bis 500 nm, vorzugsweise 20 bis 200 nm; nanopartikuläre Füllstoffe, wie Tantal(V)-oxid, Bariumsulfat oder Mischoxide von SiO₂ mit Ytterbium(III)-oxid oder Tantal(V)-oxid mit einer durchschnittlichen Teilchengröße von 5 bis 500 nm, vorzugsweise 20 bis 200 nm.

Neben den genannten Füllstoffen können die Werkstoffe Füllstoffe mit einer Partikelgröße von vorzugsweise < 50 nm, besonders bevorzugt < 40 nm enthalten. Die Partikelgröße liegt vorzugsweise im Bereich von 10-50 nm und besonders bevorzugt von 10-40 nm. Diese Füllstoffe streuen aufgrund ihrer geringen Partikelgröße sichtbares Licht nicht und haben damit keinen Einfluss auf die Transluzenz. Bevorzugte Beispiele für diese Füllstoffe sind pyrogene Kieselsäure und Fällungskieselsäure.

Bei allen Partikelgrößen handelt es sich um Gewichtsmittel. Die Lichtstreuung nimmt mit abnehmender Partikelgröße ab, jedoch haben Füllstoffe mit geringer Partikelgröße eine größere Verdickungswirkung. Erfindungsgemäß sind daher Füllstoffe mit einer Partikelgröße im Bereich von 100 nm bis 5 µm und insbesondere im Bereich von 200 nm bis 2 µm bevorzugt.

Die Füllstoffe sind vorzugsweise oberflächenmodifiziert, besonders bevorzugt durch Silanisierung, insbesondere mit 3-Methacryloyloxypropyltrimethoxysilan. Die Silanisierung hat keinen messbaren Einfluss auf den Brechungsindex des Füllstoffs.

Besonders bevorzugt sind Dentalmaterialien, die als Füllstoff ein Barium- oder Strontiumaluminiumborsilikatglas, pyrogene Kieselsäure, ein Mischoxid aus SiO₂ und ZrO₂, oder Ytterbiumfluorid oder eine Mischung davon enthalten.

Gegebenenfalls können die erfindungsgemäß eingesetzten Zusammensetzungen weitere Additive enthalten, vor allem Stabilisatoren, wie z.B. Polymerisationsstabilisatoren, Farbmittel, antibakterielle Wirkstoffe, fluoridionenabgebende Additive, optische Aufheller, Fluoreszenzmittel, UV-Absorber, Stoffe zur Verbesserung der Bruchzähigkeit und/oder Effektmittel.

Vorzugsweise enthalten die erfindungsgemäßen Materialen mindestens eine basische Komponente, vorzugsweise ein tertiäres Amin als Stabilisator. Diese verhindert eine vorzeitige spontane kationische Polymerisation der π-elektronenreichen Vinylsulfidgruppen der difunktionellen Phenylensulfide der allgemeinen Formel I. Die basische Komponente wird vorzugsweise in einer Menge von 10 bis 1000 ppm zugesetzt.

Erfindungsgemäß sind radikalisch polymerisierbare Dentalwerkstoffe bevorzugt, die
(a) mindestens ein difunktionelles Phenylensulfid gemäß der Formeln I,
(b) mindestens einen Photoinitiator für die radikalische Polymerisation,
(c) mindestens ein weiteres radikalisch polymerisierbares Monomer, und
(d) vorzugsweise mindestens einen Füllstoff enthalten.

Besonders bevorzugt sind Dentalmaterialien, die
(a) 0,1 bis 95 Gew.-%, bevorzugt 1 bis 80 Gew.-% und besonders bevorzugt 5 bis 70 Gew.-% difunktionelle Phenylensulfide der Formeln I,
(b) 0,01 bis 5 Gew.-%, besonders bevorzugt 0,01 bis 3,0 Gew.-% und besonders bevorzugt 0,1 bis 1,0 Gew.-% Initiator,
(c) 1 bis 80 Gew.-%, bevorzugt 1 bis 60 Gew.-% und besonders bevorzugt 10 bis 50 Gew.-% weiteres radikalisch polymerisierbares Monomer, und
(d) 0 bis 85 Gew.%, bevorzugt 10 bis 85 Gew.-% und besonders bevorzugt 30 bis 85 Gew.-% Füllstoff enthalten.

Der Füllungsgrad richtet sich nach dem gewünschten Anwendungszweck des Werkstoffs. Füllungskomposite haben vorzugsweise einen Füllstoffgehalt von 25-85 Gew.-% und Kompositzemente von 25-70 Gew.-%.

Alle Mengenangaben (Gew.-% und ppm) hierin beziehen sich auf die Gesamtmasse des Dentalwerkstoffs, wenn nicht anders angegeben.

Besonders bevorzugt sind solche Dentalwerkstoffe, die aus den genannten Stoffen bestehen. Weiterhin sind solche Werkstoffe bevorzugt, bei denen die einzelnen Komponenten jeweils aus den oben genannten bevorzugten und besonders bevorzugten Stoffen ausgewählt sind.

Die erfindungsgemäßen Dentalmaterialien enthalten als radikalisch polymerisierbares Monomer (c) Bis-GMA, UDMA, TMX-UDMA, D₃MA, TEGDMA, CMP-1E, TCP, SR-348c oder eine Mischung davon. Außerdem bevorzugt sind Dentalmaterialien, die als Füllstoff (d) ein Barium- oder Strontiumaluminiumborsilikatglas, pyrogene Kieselsäure, ein Mischoxid aus SiO₂ und ZrO₂, oder Ytterbiumfluorid oder eine Mischung davon enthalten.

Die erfindungsgemäßen Werkstoffe eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Die Dentalwerkstoffe eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken.

Die Erfindung wird im Folgenden anhand von Beispielen näher erläutert.

### Ausführungsbeispiele

### Beispiel 1:

### Synthese von Bis(4-vinylthiophenyl)sulfid

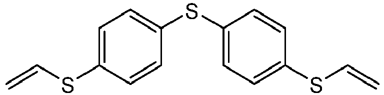

Zur Synthese von Bis(4-vinylthiophenyl)sulfid wurde 4,4-Thiobis(benzolthiol) zunächst mit einem Überschuss an 1,2-Dichlorethan in Gegenwart von KOH und Tetrabutylammoniumchlorid bei Raumtemperatur (23°C) umgesetzt. Das dabei erhaltene Bis[4-(2-chlorethylthiophenyl)sulfid wurde mit KOH in Wasser bei 85 °C zu Bis(4-vinylthiophenyl)sulfid dehydrohalogeniert. Bis(4-vinylthiophenyl)sulfid wurde als bei Raumtemperatur farblose, niedrig viskose Flüssigkeit mit einer Viskosität η von nur 17 mPa s (23 °C) erhalten. Der Brechungsindex n_{D} beträgt 1,695 und der Polymerisationsschrumpf Δ_{P}V -7,8 Vol.-%. Der Polymerisationsschrumpf wurde nach der DIN 13907: "Zahnheilkunde: Polymerisationschrumpfung von Füllungswerkstoffen" gemessen.

### Beispiel 2:

### Monomermischungen mit einem erfindungsgemäßen Monomer

Ausgehend von Bis(4-vinylthiophenyl)sulfid und dem häufig eingesetzten dentalen Verdünnermonomer Triethylenglycoldimethacrylat (TEGDMA) wurden verschiedene Monomermischungen hergestellt und deren Brechungsindices bestimmt (Tabelle 1). Die Eigenschaften von TEGDMA sind: η = 10 mPa s (23 °C), n_{D} = 1,461 und Δ_{P}V = -14,3 Vol.-%.

Die Ergebnisse in Tabelle 1 belegen, dass das erfindungsgemäße Monomer sehr gut dazu geeignet ist, um ausgehend von üblichen dentalen Verdünnermonomeren, die einen niedrigen Brechungsindex zeigen, Mischungen mit deutlich höherem Brechungsindex herzustellen.

**Tabelle 1: Brechungsindices von Mischungen aus Bis(4-vinylthiophenyl)sulfid und TEGDMA**

| **Monomermischung [Gew.-%/Gew.-%]** | **Brechungsindex** |
|---|---|
| TEGDMA | 1,461 |
| Bis(4-vinylthiophenyl)sulfid / TEGDMA: 10:90 | 1,482 |
| Bis(4-vinylthiophenyl)sulfid / TEGDMA: 20:80 | 1,504 |
| Bis(4-vinylthiophenyl)sulfid / TEGDMA: 40:60 | 1,548 |
| Bis(4-vinylthiophenyl)sulfid / TEGDMA: 50:50 | 1,572 |
| Bis(4-vinylthiophenyl)sulfid | 1,695 |

### Beispiel 3:

### Lichthärtendes Harz

Zur Herstellung eines lichthärtenden Harzes wurden 5,0 g Bis(4-vinylthiophenyl)sulfid mit 4,95 g TEGDMA und dem Photoinitiator Ivocerin® (Bis(4-methoxybenzoyl)diethylgermanium (Ivoclar Vivadent AG), 50 mg) gemischt. Von der Mischung wurden entsprechende Prüfkörper präpariert, die 2 mal 3 Minuten mit einer dentalen Lichtquelle (Spectramat®, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit (**BF**) und des Biege-E-Moduls (**BM**) nach 24 h Lagerung der Prüfkörper bei Raumtemperatur (**RT**) oder 24 h bzw. 1 Woche (7 d) Lagerung in Wasser bei 37 °C (**WL**) (Tabelle 2):

**Tabelle 2: Mechanische Eigenschaften einer Mischung von Bis(4-vinyl-thiophenyl)sulfid und TEGDMA nach Lichthärtung**

| | **24 h RT** | **24 h WL** | **7 d WL** |
|---|---|---|---|
| **BF (MPa)** | 82,4±10,3 | 96,9±13,3 | 78,6±9,0 |
| **BM (GPa)** | 2,36±0,21 | 2,62±0,19 | 2,71±0,15 |

Die Ergebnisse der mechanischen Untersuchungen in Tabelle 2 zeigen, dass sich das erfindungsgemäße Monomer Bis(4-vinylthiophenyl)sulfid überraschend gut mit einem Dimethacrylat radikalisch copolymerisieren lässt. Es war nicht zu erwarten, dass sich π-elektronenreiche difunktionelle Phenylensulfide der Formel I radikalisch mit Methacrylaten copolymerisieren lassen.

### Beispiel 4:

### Licthärtendes Komposit

Aus folgenden Komponenten wurde ein Komposit hergestellt: 17,60 g Bis(4-vinylthio-phenyl)sulfid, 17,42 g TEGDMA, 0,18 g Ivocerin® (Fa. Ivoclar Vivadent AG, Liechtenstein), 44,80 g der silanisierten pyrogenen Kieselsäure OX-50 (Fa. Degussa AG, Deutschland) und 20,00 g Ytterbiumtrifluorid YbF₃ (Fa. Sukgyung, Südkorea). Von den farblosen Pasten wurden analog Beispiel 3 entsprechende Prüfkörper präpariert, ausgehärtet und die Biegefestigkeit und der E-Modul bestimmt (Tabelle 3).

**Tabelle 3: Mechanische Eigenschaften eines lichthärtenden Komposits aus Bis(4-vinylthiophenyl)sulfid, TEGDMA und dentalen Füllstoffen.**

| | **24 h RT** | **24 h WL** | **7 d WL** |
|---|---|---|---|
| **BF (MPa)** | 113,1±7,7 | 107,3±16,0 | 109,5±10,3 |
| **BM (GPa)** | 7,81±0,44 | 7,31±0,65 | 7,62±0,48 |

Die Ergebnisse in Tabelle 3 belegen, dass mit dem erfindungsgemäßen Bis(4-vinyl-thiophenyl)sulfid in Mischung mit dem Dimethacrylat-Verdünner TEGDMA dentale lichthärtende Komposite mit sehr guten mechanischen Eigenschaften hergestellt werden können. Dies ist ein Beleg dafür, dass sich die π-elektronenreichen difunktionellen Phenylensulfide der Formel I auch in Anwesenheit von Füllstoff sehr gut radikalisch mit Methacrylaten copolymerisieren lassen.

## Patentansprüche

1. Extraorale Verwendung einer Zusammensetzung, die mindestens ein radikalisch polymerisierbares, difunktionelles Phenylensulfid gemäß der Formeln I, 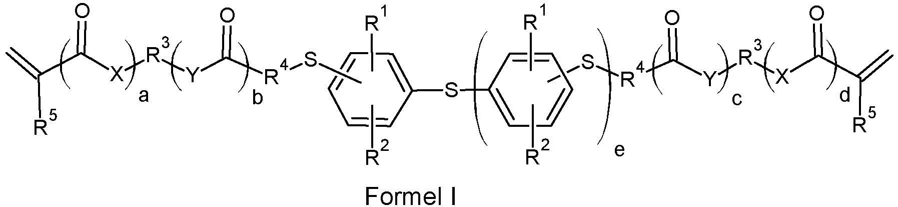 in der
X, Y unabhängig voneinander jeweils entfallen oder O oder NR⁶ sind,
R¹, R² unabhängig voneinander jeweils H, ein linearer oder verzweigter C₁- bis C₁₀-Alkylrest, -OR⁷, -SR⁸, Cl oder Br sind,
R³ entfällt oder ein C₁-C₁₀-Alkylenrest ist, der durch O oder S unterbrochen sein kann,
R⁴ entfällt oder ein C₁-C₁₀-Alkylenrest ist, der durch O oder S unterbrochen sein kann,
R⁵ H oder ein C₁-C₅-Alkylrest ist,
R⁶, R⁷, R⁸ unabhängig voneinander jeweils H oder ein linearer oder verzweigter C₁- bis C₁₀- Alkylrest sind,
a, b, c, d unabhängig voneinander jeweils 0 oder 1 sind und
e 1 oder 2 ist, und
mindestens ein weiteres radikalisch polymerisierbares Monomer ausgewählt aus 2,2-Bis[4-(2-hydroxy-3-methacryloyloxypropyl)phenyl]propan (Bis-GMA), UDMA (ein Additionsprodukt aus 2-Hydroxyethylmethacrylat und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat), Decandiol-1,10-dimethacrylat (D₃MA), Triethylenglycoldimethacrylat (TEGDMA), p-Cumylphenoxyethylenglycolmethacrylat (CMP-1E), Bis(3-methacryloyloxymethyl)tricyclo-[5.2.1.0^{2,6}]decan (TCP) und 2-[4-(2-Methacryloyloxyethoxyethoxy)phenyl]-2-[4-(2-methacryloyloxyethoxy)phenyl]propan) enthält, als Dentalwerkstoff.

2. Verwendung nach Anspruch 1, wobei die Variablen die folgenden Bedeutungen haben:
X, Y unabhängig voneinander jeweils entfällt oder O,
R¹, R² unabhängig voneinander jeweils H oder Br, CH₃, SCH₃, OCH₃,
R³ entfällt oder ein C₂-C₄-Alkylenrest,
R⁴ entfällt oder ein C₁-C₃-Alkylenrest,
R⁵ H oder CH₃,
a, b, c, d unabhängig voneinander jeweils 0 oder 1, und
e 1.

3. Verwendung nach Anspruch 1, wobei die Variablen die folgenden Bedeutungen haben:
R¹, R² unabhängig voneinander jeweils H oder Br,
R³ entfällt oder ein C₂-C₄-Alkylenrest,
R⁴ entfällt oder ein C₁-C₃-Alkylenrest,
R⁵ H,
a, b, c, d jeweils 0, und
e 1.

4. Verwendung nach einem der Ansprüche 1 bis 3, wobei das difunktionelle Phenylensulfid der Formeln I eine Viskosität von ≤ 3 Pa·s, vorzugsweise 5 bis 2.000 mPa·s und besonders bevorzugt 10 bis 1.000 mPa·s aufweist, gemessen mit einem Kapillarviskosimeter bei einer Temperatur von 23°C.

5. Verwendung nach einem der Ansprüche 1 bis 4, wobei das difunktionelle Phenylensulfid der Formeln I einen Brechungsindex von mehr 1,57, vorzugsweise von 1,575 bis 1,75 und besonders bevorzugt von 1,58 bis 1,72 aufweist, gemessen bei 20°C mit dem Licht der gelben Na-D-Linie (λ = 589 nm).

6. Verwendung nach einem der Ansprüche 1 bis 5, wobei die Zusammensetzung zusätzlich mindestens einen Initiator für die radikalische Polymerisation, vorzugsweise einen Photoinitiator enthält.

7. Verwendung nach einem der Ansprüche 1 bis 6, wobei die Zusammensetzung zusätzlich mindestens einen organischen, anorganischen oder organisch-anorganische Füllstoff enthält.

8. Verwendung nach Anspruch 7, wobei die Zusammensetzung als zusätzlichen Füllstoff ein Barium- oder Strontiumaluminiumborsilikatglas, pyrogene Kieselsäure, ein Mischoxid von SiO₂ und ZrO₂, oder Ytterbiumfluorid oder eine Mischung davon enthält.

9. Verwendung nach einem der Ansprüche 1 bis 8, wobei die Zusammensetzung
(a) 0,1 bis 95 Gew.-%, bevorzugt 1 bis 80 Gew.-% und besonders bevorzugt 5 bis 70 Gew.-% mindestens eines difunktionellen Phenylensulfids gemäß der Formel I,
(b) 0,01 bis 5 Gew.-%, bevorzugt 0,01 bis 3,0 Gew.-% und besonders bevorzugt 0,1 bis 3,0 Gew.-% mindestens eines Initiators für die radikalische Polymerisation,
(c) 1 bis 80 Gew.-%, bevorzugt 1 bis 60 Gew.-% und besonders bevorzugt 10 bis 50 Gew.-% mindestens eines weiteren radikalisch polymerisierbaren Monomers enthält, jeweils bezogen auf die Gesamtmasse des Werkstoffs.

10. Verwendung nach Anspruch 9, wobei die Zusammensetzung zusätzlich
(d) 0 bis 85 Gew.-%, bevorzugt 10-85 Gew.-% oder 30-85 Gew.-% Füllstoff enthält.

11. Zusammensetzung wie in einem der Ansprüche 1 bis 10 definiert zur therapeutischen Verwendung als dentaler Zement, Füllungskomposit oder Verblendmaterial.

12. Verwendung gemäß einem der Ansprüche 1 bis 10 zur extraoralen Herstellung oder Reparatur von Dentalrestaurationen, Prothesen, künstlichen Zähnen, Inlays, Onlays, Kronen oder Brücken.

## Claims

1. Extraoral use of a composition, wherein said composition comprises as a dental material at least one radically polymerisable, difunctional phenylene sulfide according to Formula I, 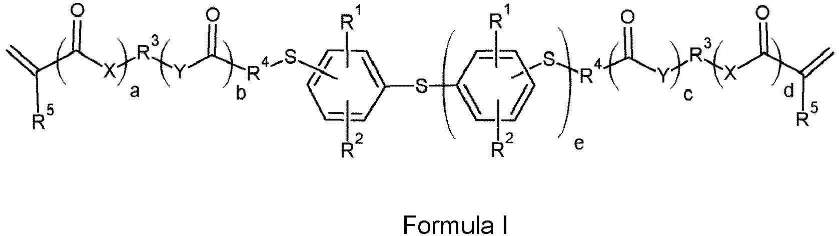 in which
X, Y independently of each other in each case are absent or are O or NR⁶,
R¹, R² independently of each other in each case are H, a linear or branched C₁-C₁₀ alkylene moiety, -OR⁷, -SR⁸, Cl or Br,
R³ is absent or is a C₁-C₁₀ alkylene moiety that can be interrupted by O or S,
R⁴ is absent or is a C₁-C₁₀ alkylene moiety that can be interrupted by O or S,
R⁵ is H or a C₁-C₅ alkylene moiety,
R⁶, R⁷, R⁸ independently of each other in each case are H or a linear or branched C₁-C₁₀ alkylene moiety,
a, b, c, d independently of each other in each case are 0 or 1 and
e is 1 or 2, and
at least one additional radically polymerisable monomer selected from 2,2-bis[4-(2-hydroxy-3-methacryloyloxypropyl)phenyl]propane (bis-GMA), UDMA (an addition product of 2-hydroxyethyl methacrylate and 2,2,4-trimethylhexamethylene diisocyanate), TMX-UDMA (an addition product of a mixture of HEMA and hydroxypropyl methacrylate (HPMA) with α,α,α',α'-tetramethyl-m-xylylene diisocyanate), 1,10-decanediol dimethacrylate (D₃MA), triethylene glycol dimethacrylate (TEGDMA), p-cumylphenoxyethylene glycol methacrylate (CMP-1E), bis(3-methacryloyloxymethyl)tricyclo-[5.2.1.0^{2,6}]-decane (TCP) and 2-[4-2-methacryloyloxyethoxyethoxy)phenyl]-2-[4-2-methacryloyloxyethoxy)phenyl]-propane.

2. Use according to claim 1, wherein the variables have the following meanings:
X, Y independently of each other in each case are absent or are O,
R¹, R² independently of each other in each case are H or Br, CH₃, SCH₃, OCH₃,
R³ is absent or is a C₂-C₄ alkylene moiety,
R⁴ is absent or is a C₁-C₃ alkylene moiety,
R⁵ is H or CH₃,
a, b, c, d independently of each other in each case are 0 or 1, and
e is 1.

3. Use according to claim 1, wherein the variables have the following meanings:
R¹, R² independently of each other in each case are H or Br,
R³ is absent or is a C₂-C₄ alkylene moiety,
R⁴ is absent or is a C₁-C₃ alkylene moiety,
R⁵ is H,
a, b, c, d are each 0, and
e is 1.

4. Use according to one of claims 1 to 3, wherein the viscosity of the difunctional phenylene sulfide of Formula I is ≤ 3 Pa·s, preferably 5 to 2 000 mPa·s and particularly preferably 10 to 1 000 mPa·s, as measured using a capillary viscometer at a temperature of 23 °C.

5. Use according to one of claims 1 to 4, wherein the refractive index of the difunctional phenylene sulfide of Formula I is greater than 1.57, preferably from 1.575 to 1.75 and particularly preferably from 1.58 to 1.72, as measured at 20 °C with the light of the yellow Na D line (λ = 589 nm).

6. Use according to one of claims 1 to 5, wherein the composition additionally comprises at least one initiator for the radical polymerisation, preferably a photoinitiator.

7. Use according to one of claims 1 to 6, wherein the composition additionally comprises at least one organic, inorganic or organic-inorganic filler.

8. Use according to claim 7, wherein the composition comprises a barium or strontium aluminium borosilicate glass, pyrogenic silica, a mixed oxide of SiO₂ and ZrO₂, or ytterbium fluoride or a mixture thereof as the additional filler.

9. Use according to one of claims 1 to 8, wherein the composition comprises
(a) 0.1 to 95 wt %, preferably 1 to 80 wt % and particularly preferably 5 to 70 wt % of at least one difunctional phenylene sulfide according to Formula I,
(b) 0.01 to 5 wt %, preferably 0.01 to 3.0 wt % and particularly preferably 0.1 to 3.0 wt % of at least one initiator for the radical polymerisation,
(c) 0 to 80 wt %, preferably 1 to 60 wt % and particularly preferably 10 to 50 wt % of at least one further radically polymerisable monomer, in each case relative to the total mass of the material.

10. Use according to claim 9, wherein the composition additionally comprises
(d) 0 to 85 wt %, preferably 10-85 wt % or 30-85 wt % filler.

11. Composition as defined in one of claims 1 to 10 for therapeutic use as a dental cement, filling composite or veneering material.

12. Use according to one of claims 1 to 10 for the extraoral production or repair of dental restorations, prosthetics, artificial teeth, inlays, onlays, crowns or bridges.

## Revendications

1. Utilisation extraorale d'une composition qui contient au moins un sulfure de phénylène difonctionnel polymérisable par voie radicalaire, de formule I : 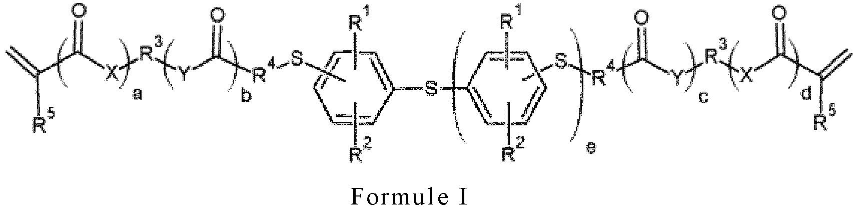 dans laquelle
- les symboles X et Y, chacun indépendamment les uns des autres, ne représentent rien ou représentent un chaînon symbolisé par O ou par NR⁶,
- les symboles R¹ et R² représentent chacun, indépendamment les uns des autres, un atome d'hydrogène, un groupe alkyle en C₁-C₁₀ linéaire ou ramifié, un groupe symbolisé par -OR⁷ ou par -SR⁸, ou un atome de chlore ou de brome,
- R³ ne représente rien ou représente un groupe alcanediyle en C₁-C₁₀, dont la chaîne peut être interrompue par un atome d'oxygène ou de soufre,
- R⁴ ne représente rien ou représente un groupe alcanediyle en C₁-C₁₀, dont la chaîne peut être interrompue par un atome d'oxygène ou de soufre,
- R⁵ représente un atome d'hydrogène ou un groupe alkyle en C₁-C₅,
- les symboles R⁶, R⁷ et R⁸ représentent chacun, indépendamment les uns des autres, un atome d'hydrogène ou un groupe alkyle en C₁-C₁₀ linéaire ou ramifié,
- les indices a, b, c et d valent chacun, indépendamment les uns des autres, 0 ou 1,
- et l'indice e vaut 1 ou 2,
et au moins un autre monomère polymérisable par voie radicalaire, choisi parmi les suivants :
- bis-GMA (2,2-bis[4-(2-hydroxy-3-méthacryloyloxy-propyl)-phényl]-propane),
- UDMA (produit d'addition de méthacrylate de 2-hydroxy-éthyle et de 2,2,4-triméthyl-hexaméthylène-diisocyanate),
- TMX-UDMA (produit d'addition d'un mélange de HEMA et de HPMA (méthacrylate d'hydroxy-propyle) et d'α,α,α',α'-tétraméthyl-méta-xylylène-diisocyanate),
- D₃MA (diméthacrylate de 1,10-décane-diol),
- TEGDMA (diméthacrylate de triéthylèneglycol),
- CMP-1E (méthacrylate de para-cumyl-phénoxy-éthylèneglycol),
- TCP (bis(3-méthacryloyloxy-méthyl)-tricyclo[5.2.1.0^{2,6}]décane),
- et 2-[4-(2-méthacryloyloxy-éthoxy-éthoxy)-phényl]-2-[4-(2-métha-cryloyloxy-éthoxy)-phényl]-propane,
en tant que matériau dentaire.

2. Utilisation conforme à la revendication 1, pour laquelle les symboles ont les significations suivantes :
- les symboles X et Y, chacun indépendamment les uns des autres, ne représentent rien ou représentent un chaînon symbolisé par O,
- les symboles R¹ et R² représentent chacun, indépendamment les uns des autres, un atome d'hydrogène ou de brome, ou un groupe symbolisé par -CH₃, -SCH₃ ou -OCH₃,
- R³ ne représente rien ou représente un groupe alcanediyle en C₂-C₄,
- R⁴ ne représente rien ou représente un groupe alcanediyle en C₁-C₃,
- R⁵ représente un atome d'hydrogène ou un groupe méthyle,
- les indices a, b, c et d valent chacun, indépendamment les uns des autres, 0 ou 1,
- et l'indice e vaut 1.

3. Utilisation conforme à la revendication 1, pour laquelle les symboles ont les significations suivantes :
- les symboles R¹ et R² représentent chacun, indépendamment les uns des autres, un atome d'hydrogène ou de brome,
- R³ ne représente rien ou représente un groupe alcanediyle en C₂-C₄,
- R⁴ ne représente rien ou représente un groupe alcanediyle en C₁-C₃,
- R⁵ représente un atome d'hydrogène,
- les indices a, b, c et d valent chacun 0,
- et l'indice e vaut 1.

4. Utilisation conforme à l'une des revendications 1 à 3, dans laquelle le sulfure de phénylène difonctionnel de formule I présente une viscosité qui, mesurée au moyen d'un viscosimètre à tube capillaire à la température de 23 °C, est inférieure ou égale à 3 Pa.s et vaut, de préférence, de 5 à 2000 mPa.s, et mieux encore, de 10 à 1000 mPa.s.

5. Utilisation conforme à l'une des revendications 1 à 4, dans laquelle le sulfure de phénylène difonctionnel de formule I présente un indice de réfraction qui, mesuré à 20 °C pour la lumière de la raie D du sodium, de couleur jaune (λ = 589 nm), est supérieur à 1,57 et vaut, de préférence, de 1,575 à 1,75, et mieux encore, de 1,58 à 1,72.

6. Utilisation conforme à l'une des revendications 1 à 5, dans laquelle la composition contient en outre au moins un amorceur de polymérisation par voie radicalaire, qui est de préférence un photo-amorceur.

7. Utilisation conforme à l'une des revendications 1 à 6, dans laquelle la composition contient en outre au moins une charge organique, inorganique ou organique-inorganique.

8. Utilisation conforme à la revendication 7, dans laquelle la composition contient, en tant que charge ajoutée, un verre de borosilicate d'aluminium et de baryum ou de strontium, de la silice pyrogénée, un oxyde mixte à base de SiO₂ et de ZrO₂, ou du fluorure d'ytterbium, ou un mélange de telles charges.

9. Utilisation conforme à l'une des revendications 1 à 8, dans laquelle la composition contient :
a) de 0,1 à 95 % en poids, de préférence de 1 à 80 % en poids, et mieux encore de 5 à 70 % en poids, d'au moins un sulfure de phénylène difonctionnel de formule I,
b) de 0,01 à 5 % en poids, de préférence de 0,01 à 3,0 % en poids, et mieux encore de 0,1 à 3,0 % en poids, d'au moins un amorceur de polymérisation par voie radicalaire,
c) et de 1 à 80 % en poids, de préférence de 1 à 60 % en poids, et mieux encore de 10 à 50 % en poids, d'au moins un autre monomère polymérisable par voie radicalaire,
étant entendu que chacun de ces pourcentages est rapporté à la masse totale du matériau.

10. Utilisation conforme à la revendication 9, dans laquelle la composition contient en outre
d) de 0 à 85 % en poids, et de préférence de 10 à 85 % en poids ou de 30 à 85 % en poids, d'une charge.

11. Composition telle que définie dans l'une des revendications 1 à 10, pour utilisation thérapeutique en tant que ciment dentaire, composite pour obturation ou matériau de placage.

12. Utilisation, conforme à l'une des revendications 1 à 10, pour la fabrication ou réparation extraorale de restaurations dentaires, de prothèses, de dents artificielles, d'incrustations de type « inlay » ou « onlay », de couronnes ou de bridges.
